# EUROPEAN PATENT SPECIFICATION

(11) **EP 1 557 182 B1**
(45) Date of publication and mention of the grant of the patent: **21.07.2010**
(21) Application number: 04257836.9
(22) Date of filing: 16.12.2004
(51) Int. Cl.: A61L 31/10, A61L 31/16, A61B 17/12

(54) **Activatable bioactive implantable medical device**
Aktivierbare bioaktive implantierbare medizinische Vorrichtung
Dispositif médical bioactif et implantable pouvant être activé

(30) Priority: 17.12.2003 US 738477; 15.06.2004 US 868303
(43) Date of publication of application: 27.07.2005
(73) Proprietor: Codman & Shurtleff, Inc., Raynham, MA 02767 (US)
(72) Inventor: Jones, Donald K., Lauderhill Florida 33351 (US); Lorenzo, Juan A., Davie Florida 33328 (US); Pomeranz, Mark L., Davie Florida33325 (US); Sherman, Darren, Ft. Lauderdale Florida 33301 (US)
(74) Representative: Tunstall, Christopher Stephen

(56) References cited:
- WO-A-00/40278
- WO-A-03/064383
- WO-A-03/092791
- US-A1- 2002 123 801
- US-A1- 2003 093 097
- US-B1- 6 299 619

## Description

### Field of the Invention

The present invention relates to a vascular occlusive device, which includes a bioactive coating placed on the device for reacting with bodily tissue in order to promote a desired result, such as for example expansion of the bioactive coating to occlude a vessel or an aneurysm.

### Description of the Prior Art

For many years medical devices, such as vascular occlusive devices, have been placed within the vasculature of the human body to occlude, or partially occlude, blood flow through the vasculature. Additionally, such devices have been introduced into aneurysms in order to fill, or partially fill, the aneurysm so as to reduce the pressure which is applied to the interior of the aneurysm in order to prevent further growth or expansion of the aneurysm. These devices may take the form of a coil, such as a helical coil, and are typically placed within the vessel or aneurysm by use of a delivery catheter which is inserted into the vessel and positioned such that the distal end of the delivery catheter is adjacent to a selected site for placement. Once the occlusive device is placed within a blood vessel or aneurysm, surrounding tissue reacts with the "foreign" object and begins to grow into and around the device to provide more complete occlusion of the vessel.

Examples of such delivery catheters are disclosed in U.S. Patent No. 5,108,407, entitled "Method And Apparatus For Placement Of An Embolic Coil" and U.S. Patent No. 5,122,136, entitled "Endovascular Electrolytically Detachable Guidewire Tip For The Electroformation Of Thrombus In Arteries, Veins, Aneurysms, Vascular Malformations And Arteriovenous Fistulas." These patents disclose catheter systems for delivering embolic coils to preselected positions within vessels of the human body in order to treat aneurysms, or alternatively, to occlude a blood vessel at a preselected location.

Occlusive devices which take the form of coils may be helically wound coils, random wound coils, coils wound within coils or other such coil configurations. Examples of various coil configurations are disclosed in U.S. Patent No. 5,334,210, entitled, "Vascular Occlusion Assembly" and U.S. Patent No. 5,382,259, entitled, "Vasoocclusion Coil With Attached Tubular Woven Or Braided Fibrous Covering." Such coils are generally formed from radiopaque metallic materials, such as platinum, gold, tungsten or alloys of these metals. Oftentimes several coils are placed at a given location within a vessel, or within an aneurysm, to more completely occlude, or partially occlude, the flow of blood through the vessel or aneurysm. Thrombus growth onto the coils further enhances the occlusive effect of the coils.

In the past, embolic coils have been placed within the distal end of a delivery catheter and when the distal end of the catheter is properly positioned, the coil may then be pushed out of the end of the catheter with, for example a guidewire, to release the coil at the desired location. This procedure of placement of the embolic coil is conducted under fluoroscopic visualization such that the movement of the coil may be monitored and the coil may be placed at a desired location.

In addition, such coils have been specifically designed to be stretch resistant, such as the vasculature occlusive coil disclosed in U.S. Patent No. 5,853,418, entitled, "Stretch Resistant Vaso-Occlusive Coils (II)" which discloses a helically wound coil having a polymeric stretch resistant member extending through the lumen of the coil and fixedly attached to both ends of the coil to prevent the coil from stretching.

In order to increase the thrombogenicity of an embolic coil, such coils have included a coating, such as collagen, which is applied to the surface of the coil. This concept is disclosed in U.S. Patent No. 5,690,671, entitled, "Embolic Elements And Methods And Apparatus For Their Delivery," which discloses such a collagen coated embolic coil.

In addition, U.S. Patent No. 5,980,550, entitled, "Water-Soluble Coating For Bioactive Vasoocclusive Devices," discloses an embolic coil having a thrombogenic inner coating which serves as a thrombogenic agent and an outer coating of a water-soluble agent which dissolves after placement of the coil in order expose the thrombogenic inner coating to enhance the growth of thrombus into and around the coil. The water-soluble coating prevents the thrombogenic inner coating from coming into contact with the surrounding blood until the water-soluble coating is dissolved by contact with blood. While the vasculature occlusive device disclosed in this patent includes an agent for enhancing thromboginicity of the device and also includes an outer coating to prevent such activity until the outer coating is dissolved by blood flow, there is no control over when the dissolving process begins and therefore no control over the time in which the thrombogenic agent becomes activated. Without such control, it is possible that thrombus can begin forming on the coil prior to the time the coil is properly placed within a vessel, or aneurysm, therefore making it very difficult if not impossible to reposition, or remove, the improperly placed coil. Additionally, with water-soluble outer protective coatings the passive process of removing the outer coating may be so slow that the reaction may not occur in a timely manner.

Still further, U.S. Patent No. 6,602,261, entitled, "Filamentous Embolic Device With Expansible Elements," discloses an embolic coil having embolizing elements placed along a filament, or coil, which are comprised of a hydrophilic, polymeric, hydrogel foam material, such as hydrogel foam. After implantation of this embolic coil within an aneurysm, the water-swellable foam begins to expand and more completely fill the aneurysm. While the expansible embolizing elements of this embolic coil, upon expansion, serve to more completely fill an aneurysm, there is again no control over when the expansible elements begin to expand. With no control over the time of expansion, the embolic coils may begin expanding prior to being properly placed within an aneurysm or may expand prior to the placement of multiple coils within an aneurysm thereby making it very difficult to properly place multiple coils within the aneurysm. After the expansion of the embolizing elements has occurred, it may be very difficulty, or even impossible to reposition the embolic coil.

### SUMMARY OF THE INVENTION

In accordance with the present invention, there is provided a vascular occlusion device, such as a vascular occlusive coil, which includes a support member which may take the form of a helical coil, an expandable foam embolizing element like for example a bioactive expandable foam coating which is disposed on the support member, and an outer barrier comprised of ethylene vinyl alcohol which is disposed on the expandable foam coating to prevent contact between the expandable coating and a bodily fluid when the medical device is inserted into a blood vessel or an aneurysm. The expandable coating preferably takes the form of a hydrophilic, polymeric material, such as hydrogel. The outer barrier exhibits the characteristic of being non-water soluble, but dissolves upon being exposed to an external liquid agent. The external agent takes the form of a liquid medium which may be injected through a catheter into the blood vessel or aneurysm.

In accordance with the present invention, the expansible foam coating takes the form of a coating of a hydrophilic foam material, which is applied to the support member and which serves to expand upon contact with bodily fluids, such as blood, to thereby enhance the embolizing effect of the medical device. The expandable foam coating preferably takes the form of a hydrophilic, polymeric foam material, such as hydrogel foam. The outer barrier comprised of ethylene vinyl alcohol takes the form of an outer coating applied to the expansible coating and prevents bodily fluid from reacting with the expansible coating until such time as the outer barrier is exposed to an external liquid agent. The external agent may take the form of a solvent which when applied to the outer barrier through a catheter from a source outside of the body causes the outer barrier to dissolve away from the expansible coating.

In accordance with the present invention, there is provided a vascular occlusive device, which includes an expansible foam element and an outer barrier comprised of ethylene vinyl alcohol applied to the expansible foam element which prevents a reaction between bodily fluid and the expansible element until such time as an external liquid agent is applied to the outer barrier to thereby cause the outer barrier to dissolve away from the expansible element.

### BRIEF DESCRIPTION OF THE DRAWINGS

Figure 1 is an elevational view illustrating a vascular occlusive coil in accordance with one embodiment of the present invention;
Figure 2 is an elevational view, partly in cross-section illustrating the vascular occlusive coil as shown in Figure 1 illustrating a bioactive expansible coating and an outer barrier coating in accordance with the present invention;
Figure 3 is an elevational view, partly in cross-section illustrating the vascular occlusive coil as shown in Figure 2 after the outer barrier coating has been removed and the expansible coating has reacted to bodily fluid and has expanded, and,
Figures 4A through 4C illustrate the method steps of applying multiple vascular occlusive coils as shown in Figure 1 into an aneurysm and thereafter applying an external agent to thereby activate the embolic coils.

### DESCRIPTION OF THE PREFERRED EMBODIMENT

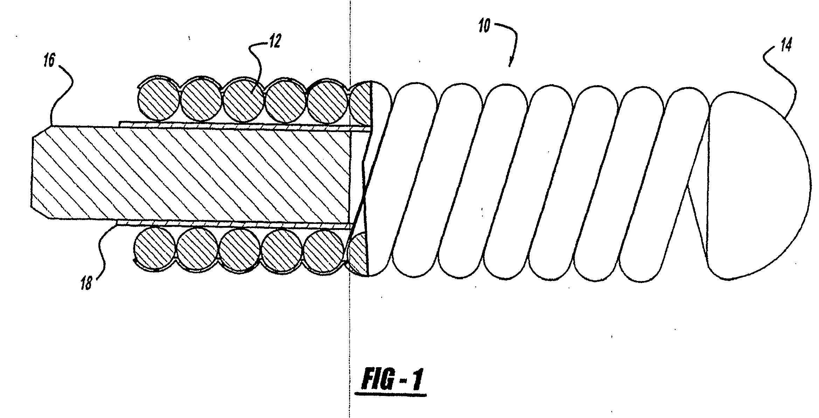

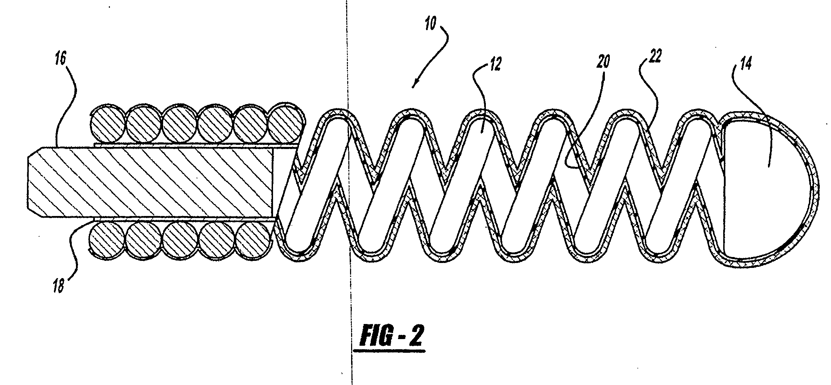

Figures 1 and 2 illustrate an embolic coil 10 which may be placed along with other similar coils into a blood vessel or into an aneurysm in order to partially fill the aneurysm. More particularly, the embolic coil 10, or vascular occlusive coil, is a typical embolic coil which comprises a helically wound coil 12 formed from a platinum alloy wire wound into a helical configuration. The diameter of the wire is generally in the range of about 18 µm (0.0007 inches) to about 20µm (0.008 inches) and the outer diameter of the coil 12 is preferably in a range of about 76µm (0.003 inches) to about 1400µm (0.055 inches). While the particular embolic coil 10 illustrated in figures 1 and 2 is shown as being a straight, helically wound coil, it should be appreciated that embolic coils are formed in various configurations and may take the form of a helical wire wound in a helical configuration, a random shaped configuration or even a coil within a coil.

Preferably the embolic coil 10 includes a weld bead 14 which is attached to the distal end of the coil for providing a less traumatic distal end for the embolic coil 10. In addition, the embolic coil 10 includes a cylindrical headpiece 16 which is placed into the lumen of the helically wound coil 12 at the proximal end of the coil and is held in place by an adhesive material 18 interposed between the cylindrical headpiece 16 and the helically wound coil 12. The construction of the embolic coil 10 and an associated hydraulic deployment system for placing the embolic coil within an aneurysm is disclosed in more detail in U.S. Patent Application Serial No. 10/102,154, entitled, "Small Diameter Embolic Coil Hydraulic Deployment System," filed March 19, 2002, assigned to the same assignee of the present invention.

Figure 2 illustrates in more detail the embolic coil 10 which comprises the helically wound coil 12, a bioactive expansible coating 20 disposed upon the coil 12, and an outer barrier 22 disposed upon the expansible coating 20 for preventing the activation of the expansible coating until such time as an election is made to activate the coating. More particularly, the expansible coating 20, may take the form of a hydrophilic, foam polymeric material, such as a hydrogel, which when exposed to bodily fluids expands. While the expansible foam coating may take the form of a hydrophilic material, it should be understood that it may take any form of any material which would expand upon reaction with bodily fluids or to other agents.

The outer barrier 22 takes the form of a non-water soluble coating which is disposed upon the bioactive expansible coating 20 and serves to insulate the expansible coating from adjacent bodily fluid until such time as a decision is made by a physician to activate the outer barrier 22 by applying an external liquid agent to the barrier. The outer barrier 22 takes the form of ethylene vinyl alcohol, a material which is inert to bodily fluid, but which dissolves and exposes the expansible coating 20 when the outer barrier 22 is subjected to an external liquid agent.

The outer barrier 22 is comprised of ethylene vinyl alcohol, and the external agent for dissolving the outer barrier 22 is comprised of dimethyl sulfoxide (DMSO) which when applied through a catheter from an external source serves to dissolve the outer barrier 22 to thereby expose the expansible foam coating 20. The expansible foam coating 20 is comprised of a hydrophilic hydrogel and in particular, a water-expansible foam matrix polymer of the type disclosed in U.S. Patent No. 5,750,585 entitled, "Super Absorbent Hydrogel Foams."

It should be appreciated that there are numerous materials which would serve as an expansible foam embolizing element, an outer barrier non-water soluble and liquid agent for dissolving or removing the outer barrier.

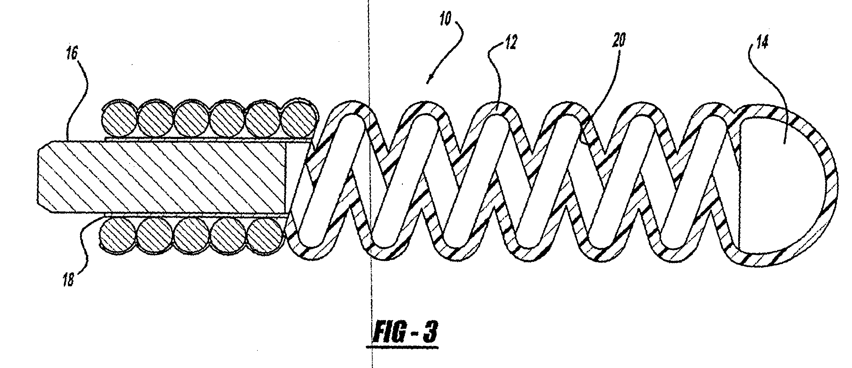

Figure 3 illustrates in more detail the embolic coil 10 after placement into an aneurysm and after the outer barrier 22 has been dissolved thereby exposing the , expansible coating 20 to bodily fluid, such as blood. As may be noted the expansible coating 20 has expanded substantially thereby causing the embolic coil 10 to more completely fill a blood vessel or an aneurysm. The outer barrier comprised of ethylene vinyl alcohol is inert to bodily fluids or blood, i.e., is not water-soluble, and the external liquid agent, or solvent, is applied through a catheter from a source outside of the body to thereby dissolve or remove the outer barrier 22 for activation of the expansible coating 20.

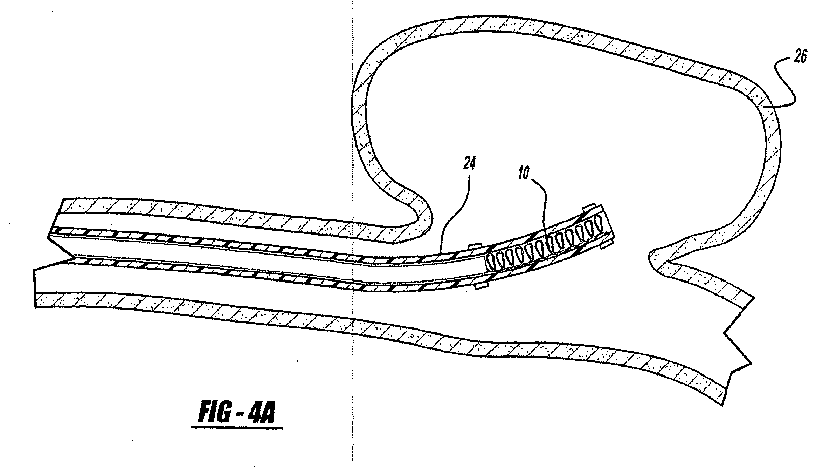

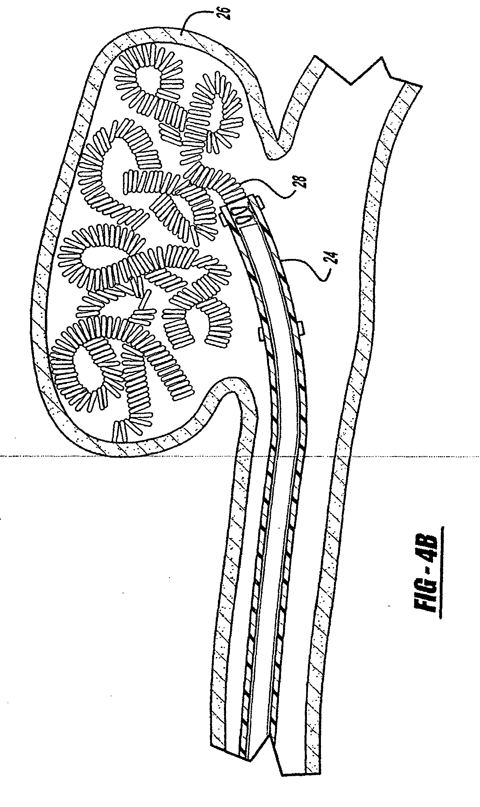

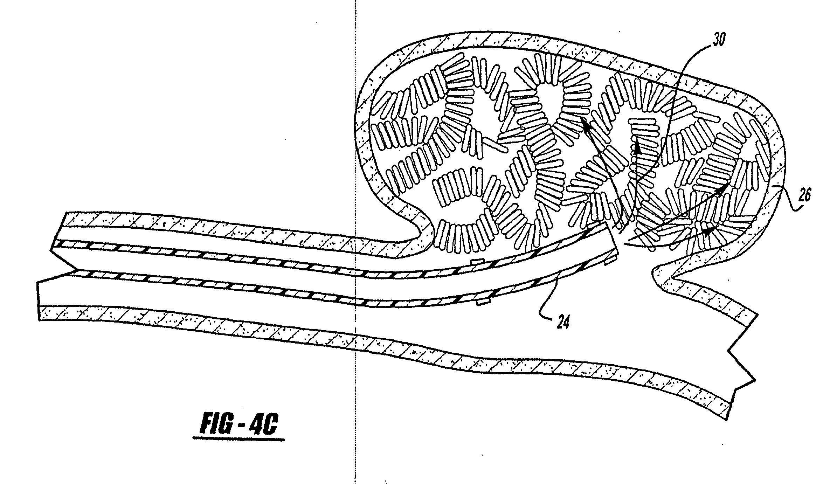

Figures 4A through 4C generally illustrate a method of utilizing the present invention. More particularly. Figure 4A illustrates a delivery catheter 24 having an embolic coil 10 placed in the distal end of the catheter for delivery into an aneurysm 26. Figure 4B illustrates the delivery catheter 24 being used to position multiple vascular occlusive coils including a final coil 28 into the aneurysm 26. Figure 4C illustrates the application of an external agent 30, which may take the form of a solvent, for dissolving the outer barrier 22, through a catheter from a source external of the body to thereby activate the expansible coating 20. The expansible coatings 20 are illustrated in their expanded configurations.

It may be desirable to place all of the vascular occlusive coils into the aneurysm 26 prior to applying the external liquid agent 30, however, another approach is that of placing a single coil into the aneurysm and thereafter activating that single coil, placing a second coil into the aneurysm and thereafter activating the second coil and so forth until all of the coils have been properly placed into the aneurysm. As may be appreciated, the advantage of the subject invention over prior devices is that the physician may determine at what point in time during the process of "filling" a blood vessel or an aneurysm the physician elects to activate the coil or coils for expansion.

## Claims

1. A vascular occlusion device comprising:
an expandable foam embolizing element; and,
an outer barrier disposed on said expandable foam embolizing element to prevent exposure of said embolizing element to bodily fluid when said vascular occlusion device is inserted into a blood vessel, said outer barrier exhibiting the characteristic of being non-water soluble but dissolving when an external activating agent is applied to said outer barrier, wherein the external agent is a liquid medium, and wherein said outer barrier is comprised of ethylene vinyl alcohol.

2. A vascular occlusion device as defined in Claim 1, wherein said expandable embolizing element is comprised of a hydrophilic foam material.

3. A vascular occlusion device as defined in Claim 2, wherein said expandable embolizing element is comprised of hydrogel foam.

4. A vascular occlusion device as defined in Claim 3, wherein the outer barrier takes the form of a coating applied to the expandable embolizing element.

5. A vascular occlusion device as defined in Claim 1, wherein said external activating agent is comprised of dimethyl sulfoxide.

6. A vascular occlusion device as defined in Claim 1, wherein said expandable foam embolizing element is disposed on a support member.

7. A vascular occlusion device as defined in Claim 6, wherein said support member comprises a helical coil.

## Patentansprüche

1. Vaskulare Verschlusseinrichtung, die aufweist:
ein embolisierendes Element aus expandierbarem Schaum; und
eine äußere Barriere, die auf dem embolisiserenden Element aus expandierendem Schaum angeordnet ist, um den Kontakt des embolisierenden Elementes mit Körperfluid zu verhindern, wenn die vaskulare Verschlusseinrichtung in ein Blutgefäß eingesetzt wird, wobei die äußere Barriere die Eigenschaft zeigt, dass sie nicht wasserlöslich ist, sich jedoch auflöst, wenn ein externes aktivierendes Mittel auf die äußere Barriere aufgegeben wird, wobei das externe Mittel ein flüssiges Medium ist und wobei die äußere Barriere aus Ethylenvinylalkohol besteht.

2. Vaskulare Verschlusseinrichtung nach Anspruch 1, bei der das expandierbare embolisierende Element aus einem hydrophilen Schaummaterial besteht.

3. Vaskulare Verschlusseinrichtung nach Anspruch 2, bei der das expandierbare embolisiserende Element aus Hydrogelschaum besteht.

4. Vaskulare Verschlusseinrichtung nach Anspruch 3, bei der die äußere Barriere die Form eines Überzuges annimmt, der auf das expandierbare embolisierende Element aufgebracht ist.

5. Vaskulare Verschlusseinrichtung nach Anspruch 1, bei der das externe aktivierende Mittel aus Dimethylsulfoxid besteht.

6. Vaskulare Verschlusseinrichtung nach Anspruch 1, bei der das expandierbare embolisierende Element aus Schaum auf einem Trägerelement angeordnet ist.

7. Vaskulare Verschlusseinrichtung nach Anspruch 6, bei der das Trägerelement eine Wendelspule aufweist.

## Revendications

1. Dispositif d'occlusion vasculaire comprenant :
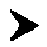 un élément embolisant en mousse expansible, et
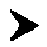 une barrière extérieure disposée sur ledit élément embolisant à mousse expansible pour empêcher l'exposition dudit élément embolisant à un fluide corporel quand ledit dispositif d'occlusion vasculaire est inséré dans un vaisseau sanguin, ladite barrière extérieure présentant la caractéristique d'être non soluble dans l'eau mais se dissolvant quand un agent activateur extérieur est appliqué à ladite barrière extérieure, dans lequel l'agent extérieur est un milieu liquide, et dans lequel ladite barrière extérieure comprend un alcool de vinyle d'éthylène.

2. Dispositif d'occlusion vasculaire selon la revendication 1, dans lequel ledit élément embolisant expansible comprend un matériau en mousse hydrophile.

3. Dispositif d'occlusion vasculaire selon la revendication 2, dans lequel ledit élément embolisant expansible comprend une mousse hydrogel.

4. Dispositif d'occlusion vasculaire selon la revendication 3, dans lequel la barrière extérieure prend la forme d'un revêtement appliqué à l'élément embolisant expansible.

5. Dispositif d'occlusion vasculaire selon la revendication 1, dans lequel ledit agent activateur externe comprend du diméthylsulfoxyde.

6. Dispositif d'occlusion vasculaire selon la revendication 1, dans lequel ledit élément embolisant en mousse expansible est disposé sur un élément de support.

7. Dispositif d'occlusion vasculaire selon la revendication 6, dans lequel ledit élément de support comprend une bobine hélicoïdale.
